(19) Europäisches Patentamt
European Patent Office
Office européen des brevets

(11) **EP 1 927 370 B1**

(12) **EUROPÄISCHE PATENTSCHRIFT**

(45) Veröffentlichungstag und Bekanntmachung des Hinweises auf die Patenterteilung:
**02.10.2013  Patentblatt 2013/40**

(51) Int Cl.:
***A61M 1/16*** *(2006.01)*

(21) Anmeldenummer: **08003419.2**

(22) Anmeldetag: **12.08.1998**

(54) **Verfahren zur Bestimmung von Parametern der Hämodialyse und Blutbehandlungsvorrichtung mit einer Einrichtung zur Bestimmung von Parametern der Hämodialyse**

Method for setting parameters for hemodialysis and blood processing device with a device for setting parameters for hemodialysis

Procédé de détermination de paramètres d'hémodialyse et dispositif de traitement sanguin à l'aide d'un dispositif destiné à déterminer des paramètres d'hémodialyse

(84) Benannte Vertragsstaaten:
**DE ES FR GB IT**

(30) Priorität: **13.08.1997  DE 19734992**
**19.02.1998  DE 19806900**

(43) Veröffentlichungstag der Anmeldung:
**04.06.2008  Patentblatt 2008/23**

(62) Dokumentnummer(n) der früheren Anmeldung(en) nach Art. 76 EPÜ:
**98115125.1 / 0 898 974**

(73) Patentinhaber: **Fresenius Medical Care Deutschland GmbH**
**61352 Bad Homburg v.d.H. (DE)**

(72) Erfinder:
• **Goldau, Rainer**
  **23847 Kastorf (DE)**
• **Krämer, Matthias**
  **61381 Friedrichsdorf (DE)**

(74) Vertreter: **Oppermann, Frank et al**
**OANDO Oppermann & Oppermann LLP**
**John-F.-Kennedy-Straße 4**
**65189 Wiesbaden (DE)**

(56) Entgegenhaltungen:
**WO-A-94/08641      DE-A1- 3 938 662**
**US-A- 4 923 613**

**Beschreibung**

Die Erfindung betrifft ein Verfahren zur Bestimmung von Parametern der Hämodialyse während einer extrakorporalen Blutbehandlung sowie eine Blutbehandlungsvorrichtung mit einer Einrichtung zur Bestimmung von Parametern der Hämodialyse.

Eine wesentliche Aufgabe der Nieren des Menschen liegt in der Absonderung harnpflichtiger Stoffe aus dem Blut und der Regelung der Wasser- und Elektrolytausscheidung. Die Hämodialyse stellt ein Behandlungsverfahren zur Kompensation von Fehlfunktionen der Nieren bezüglich der Entfernung der harnpflichtigen Stoffe und der Einstellung der Elektrolytkonzentration im Blut dar. Die Wasserausscheidung wird dabei über eine Ultrafiltrationseinrichtung kontrolliert.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration ($c_d$) der Dialysierflüssigkeit entspricht der Konzentration des Blutes eines Gesunden. Während der Behandlung wird das Blut des Patienten und die Dialysierflüssigkeit an beiden Seiten der Membran im allgemeinen im Gegenstrom mit einer vorgegebenen Flußrate ($Q_b$ bzw. $Q_d$) vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Durch Anlegen eines Transmembrandrucks kann der Stoffwechsel zusätzlich beeinflußt werden.

Um das Blutbehandlungsverfahren optimieren zu können, ist die Bestimmung von Parametern der Hämodialyse während der extrakorporalen Blutbehandlung (in-vivo) notwendig. Von Interesse ist insbesondere der Wert für die Konzentration einer Substanz im Blut am Eingang des Dialysators oder die Austauschleistung des Dialysators für eine bestimmte Substanz, die durch die sogenannte "Clearance" bzw. "Dialysance D" dargestellt wird.

Als Clearance für einen bestimmten Stoff K wird dasjenige virtuelle (errechnete) Blutvolumen bezeichnet, das pro Minute unter definierten Bedingungen im Dialysator vollkommen von einem bestimmten Stoff befreit wird. Die Dialysance ist ein weiterer Begriff zur Bestimmung der Leistungsfähigkeit eines Dialysators, bei dem die Konzentration der eliminierten Substanz in der Dialysierflüssigkeit berücksichtigt wird. Neben diesen Parametern zur Beschreibung der Leistungsfähigkeit des Dialysators sind noch andere Parameter von Bedeutung, wie die Werte des wäßrigen Anteils des Blutes, des Blutvolumens und der Bluteingangskonzentration etc..

Die meßtechnisch-mathematische Quantifizierung der Blutreinigungsverfahren und die Bestimmung der vorgenannten Parameter der Dialyse ist relativ komplex. Hinsichtlich der Berechnungsgrundlagen wird auf Sargent" J.A., Gotch. F.A.,: Principles and biophysics of dialysis, in: Replacement of Renal Function by Dialysis, W. Drukker, F.M. Parsons, J.F. Maher (Hrsg). Nijhoff, Den Haag 1983 verwiesen.

Die Dialysance bzw. die Clearance kann für einen gegebenen Elektrolyten, beispielsweise Natrium, bei einer Ultrafiltrationsrate von Null wie folgt bestimmt werden. Die Dialysance D ist gleich dem Verhältnis zwischen dem blutseitigen Massentransport für diesen Eletkrolyten ($Q_b$ x (cbi - cbo)) und der Konzentrationsdifferenz dieses Elektrolyten zwischen dem Blut und der Dialysierflüssigkeit am jeweiligen Eingang des Dialysators (cbi - cdi).

$$D = Qb \bullet \frac{cbi - cbo}{cbi - cdi} \qquad (1)$$

Aus Gründen der Massenbilanz ilt:

$$Qb \bullet (cbi - cbo) = -Qd \bullet (cdi - cdo) \qquad (2)$$

Aus den beiden oben genannten Gleichungen (1) und (2) folgt:

$$D = Qd \bullet \frac{cdo - cdi}{cbi - cdi} \qquad (3)$$

Dabei bedeuten in (1) bis (3):

$Q_b$ = effektiver Blutfluß
$Q_d$ = Dialysierflüssigkeitsfluß
cb = Stoffkonzentration im Blut
cd = Stoffkonzentration in der Dialysierflüssigkeit
i = Eingang des Dialysators
o = Ausgang des Dialysators

Der effektive Blutfluß ist der Fluß des Blutanteils, in dem die zu entfernenden Stoffe gelöst sind, d.h., er bezieht sich auf das (wäßrige) Lösungsvolumen für diesen Stoff. Je nach Stoff kann das der Plasmawasserfluß oder der Blutwasserfluß, d.h. der gesamte Wasseranteil im Vollblut sein. Die Konzentration cb bezieht sich auf den entsprechenden Blutanteil.

Für den Fall eines besonderen Stoffwechselausscheidungsprodukts, z.B. Harnstoff, ist cdi = 0, da diese Substanz bestimmungsgemäß in der frischen Dialysierflüssigkeit nicht vorhanden ist.

Dann spricht man nicht mehr von der Dialysance, sondern vor der Clearance K für dieses Stoffwechselprodukt.

$$K = Qb\frac{(cbi - cbo)}{cbi} = Qd\frac{cdo}{cbi} \qquad (4)$$

Die bekannten Verfahren zur in-vivo-Bestimmung von Parametern der Hämodialyse basieren auf den obigen Überlegungen. Dabei besteht das Bestreben, ohne einen direkten Meßeingriff in die Blutseite auszukommen, da dieser nämlich eine nicht unerhebliche Gefahrenquelle darstellt. Die zu bestimmenden Größen müssen daher allein aus dialysatseitigen Messungen abgeleitet werden.

Durch die EP 0 291 421 B1 ist ein Verfahren zur Bestimmung der Bluteingangskonzentration cbi bekannt geworden, bei dem rampenförmig die Dialysateingangskonzentration verändert wird, um den Punkt zu bestimmen, wo kein Elektrolyttransfer über die Membran mehr stattfindet. Das bekannte Verfahren arbeitet daher nach dem Prinzip, die Eingangsleitfähigkeit der Dialysierflüssigkeit soweit zu verändern, daß sie sich nicht mehr von der Ausgangsleitfähigkeit unterscheidet. Dann muß sie die Bluteingangsleitfähigkeit angenommen haben (cbi = cdi). Auf der Basis der Gleichungen (1) bis (3) lassen sich dann andere Parameter der Hämodialyse ableiten wie der Wert von D oder Qb. Nachteilig bei diesem Verfahren ist die verhältnismäßig lange Meßzeit, bedingt durch die Zeitspanne bis zum Erreichen des stabilen Gleichgewichtszustandes beim Einstellen der Dialysierflüssigkeit auf den neuen Eingangskonzentrationswert, der sich zudem nicht sofort an jedem Punkt des Dialysators auswirkt. Es dauert systembedingt eine gewisse Zeit, bis ein Leitfähigkeitssprung am Dialysateingang zu stabilen Verhältnissen am Dialysatausgang führt. Die zum Erreichen des stabilen Gleichgewichtszustandes erforderliche Zeitspanne ist dabei im wesentlichen bestimmt von der Größe der Leitfähigkeitsveränderung pro Zeit. Innerhalb dieser langen Zeitspanne können sich jedoch Parameter der Dialyse ändern und somit den zu bestimmenden Wert verfälschen. Insbesondere ist zu beachten, daß das vorgenannte bekannte Verfahren (wie alle anderen auch) direkt die Bluteingangskonzentration cbi durch den herbeigeführten Elektrolyttransfer verändern kann. Im bekannten Fall ist dieser systematische Fehler durch die Art der Veränderung der Konzentration auf der Dialysatseite besonders groß. Das bekannte Verfahren führt damit nicht zu genauen Meßwerten für die in-vivo zu bestimmenden Parameter der Hämodialyse. Hinzu kommt, daß eine relativ aufwendige zusätzliche Vorrichtung zum Variieren der Dialysateingangskonzentration benötigt wird.

Die DE 39 38 662 C2 (EP 0 428 927 A1) beschreibt ein Verfahren zur in-vivo-Bestimmung von Parametern der Hämodialyse, bei dem der Dialysat-Elektrolyttransfer jeweils bei zwei unterschiedlichen Dialysateingangskonzentrationen gemessen wird. Unter der Annahme, daß die Bluteingangskonzentration konstant ist, wird nach dem bekannten Verfahren die Dialysance dadurch bestimmt, daß die Differenz zwischen den Differenzen der Dialysierflüssigkeits-Ionenkonzentration an der Eingangsseite und der Ausgangsseite des Dialysators zum Zeitpunkt der ersten und zweiten Messung bestimmt wird, diese durch die Differenz der Dialysierflüssigkeits-Ionenkonzentration an der Eingangsseite zum Zeitpunkt der ersten Messung und der zweiten Messung geteilt wird und der Quotient hieraus mit dem Dialysierflüssigkeitsfluß multipliziert wird.

Die o.g. Verfahren erweisen sich insofern als nachteilig, als relativ aufwendige Vorrichtungen zum Variieren der Dialysateingangskonzentration erforderlich sind.

Die EP 0 330 892 A1 und die EP 0 547 025 beschreiben ein weiteres Verfahren, das die Bestimmung der Bluteingangskonzentration zum Ziel hat. Bei dem bekannten Verfahren wird die Differenz der Dialysierflüssigkeits-Ionenkonzentration an der Ein- und Ausgangsseite des Dialysators, d.h. die Elektrolyttransferrate, bestimmt. Das bekannte Verfahren arbeitet auch mit unterschiedlichen Konzentrationseinstellungen im Dialysierflüssigkeitsweg, so daß ebenfalls eine relative aufwendige Vorrichtung zum Variieren der Dialysierflüssigkeitseingangskonzentration erforderlich ist.

Aus der US- A- 5, 110, 477 ist ein Verfahren zur Bestimmung der Clearance eines Dialysators bekannt, bei dem sowohl auf der Dialysat- als auch der Blutseite Kalibrierlösungen durch den Dialysator geleitet werden. Auch dieses Verfahren erfordert eine relativ aufwendige Apparatur. Darüber hinaus kann es nicht in- vivo, sondern nur in- vitro, d.h. außerhalb der laufenden Dialysebehandlung, durchgeführt werden.

Die WO 94/09351 beschreibt einen Harnstoffsensor für eine Hämodialysevorrichtung, der zur Analyse dem Dialysierflüssigkeitsweg verbrauchte Dialysierflüssigkeit entnimmt. Die Dialysierflüssigkeitsentnahme setzt eine bestimmte Flußrate der Dialysierflüssigkeit voraus.

Aus dem Artikel Clinical Validation of a Predictive Modeling Equation for Sodium, Autoren: Nguyen-Khoa Man et al.; Herausgeber: Artificial Organs, 9(2): 150 bis 154 (1985), Raven Press, New York ist ein Verfahren zum Bestimmen der Na-Konzentration im Blutkreislauf einer künstlichen Niere bekannt, wobei die Messung im Dialysierflüssigkeitskreislauf erfolgt. Bei dem bekannten Verfahren wird die zum Dialysator führende und vom Dialysator abgehende Leitung des Dialysierflüssigkeitskreislaufs kurzgeschlossen, damit nur eine kleine Menge an Dialysierflüssigkeit von der Dialysierflüssigkeitspumpe durch den Dialysator gepumpt wird. Dadurch wird erreicht, daß sich ein Gleichgewichtszustand einstellt und die Konzentrationswerte im Blutkreislauf denjenigen im Dialysierflüssigkeitskreislauf entsprechen.

Aus der EP 0 578 585 B1 ist ebenfalls ein Verfahren bekannt, bei dem die Messung auf der Dialysierflüssigkeitsseite nach der Einstellung eines Gleichgewichtszustandes erfolgt. Auch bei diesem Verfahren rezirkuliert eine nur kleine Menge an Dialysierflüssigkeit durch den Dialysator, bis sich der Gleichgewichtszustand eingestellt hat.

Die o.g. Verfahren, bei denen während der Messung nur eine kleine Menge an Dialysierflüssigkeit durch den Dialysator geleitet wird, sind insofern nachteilig, als eine Umleitung der Dialysierflüssigkeit im Dialysierflüssigkeitsweg erforderlich ist. Dies ist mit einem zusätzlichen apparativen Aufwand verbunden.

Darüber hinaus sind Hämodialysevorrichtungen bekannt, bei denen stromauf und stromab der Dialysierflüssigkeitskammer des Dialysators Absperrorgane und eine den Strömungsweg durch die Dialysierflüssigkeitskammer des Dialysators überbrückende Bypaßleitung vorgesehen sind, um eine Leckprüfung des Dialysators vornehmen zu können.

Die WO 94/08641 beschreibt ein Überwachungssystem für eine Hämodialysevorrichtung mit einem Harnstoffsensor, der im Dialysierflüssigkeitsweg angeordnet ist. Die Druckschrift schlägt vor, den Eingang und Ausgang der Dialysierflüssigkeitskammer des Dialysators über einen Bypaß für eine vorbestimmte Zeitdauer von etwa 5 min. kurzzuschließen, so daß der Dialysierflüssigkeitsfluß durch die Dialysierflüssigkeitskammer unterbrochen ist. Obwohl der Dialysierflüssigkeitsfluß unterbrochen ist, soll die Ultrafiltration hingegen aufrechterhalten werden. Nach Ablauf der vorbestimmten Zeitdauer wird dann am Ausgang der Dialysierflüssigkeitskammer eine Probe genommen, die zur Bestimmung der Clearance des Dialysators herangezogen werden kann.

Der Erfindung liegt die Aufgabe zugrunde, ein Verfahren zur in-vivo-Bestimmung von Pararmetem der Hämodialyse anzugeben, das eine sehr einfache und schnelle dialysatseitige Messung der Blutkonzentration der im Stoffaustausch des Dialysators teilnehmenden Substanz unmittelbar als der zu bestimmende Parameter oder Zwischenwert für den zu bestimmenden Parameter erlaubt ist, ohne daß der Patient während der Messung einer möglichen Belastung durch einen Flüssigkeitsentzug ausgesetzt ist.

Die Lösung dieser Aufgabe erfolgt erfindungsgemäß mit den im Patentanspruch 1 angegebenen Merkmalen.

Bei dem erfindungsgemäßen Verfahren wird der Dialysierflüssigkeitsfluß durch die zweite Kammer des Dialysators unterbrochen. Hierzu ist es nicht erforderlich, daß der Dialysator vollständig von dem Dialysierflüssigkeitskreis abgetrennt wird. Es ist beispielsweise auch möglich, nur den Zufluß in den Dialysator zu unterbrechen.

Es hat sich überraschenderweise gezeigt, daß sich ein Gleichgewichtszustand zumindest in einem Teilvolumen der Dialysierflüssigkeitskammer allein aufgrund von Diffusionseffekten auch dann relativ schnell einstellt, wenn die zweite Kammer des Dialysators während der Messung ein vollständig abgeschlossenes Volumen bildet, d.h. kein Ultrafiltrat aus der Dialysierflüssigkeitskammer abgezogen wird. Da dem Patienten während der Messung Flüssigkeit nicht entzogen wird, stellt das erfindungsgemäße Verfahren keine Belastung for einen Patienten dar, bei dem eine Ultrafiltration nicht durchgeführt werden soll.

Es ist ausreichen, wenn sich ein Gleichgewicht in einem Teilvolumen der Dialysierflüssigkeitskammer eingestellt hat, z.B. nahe am Ausgang des Dialysators, wo sich das Gleichgewicht zuerst einstellt.

Ein weiterer Vorteil des erfindungsgemäßen Verfahrens liegt darin, daß die zur Unterbrechung des Zuflusses bzw. Abflusses der Dialysierflüssigkeit erforderlichen Absperrorgane und die für die Überbrückung des Strömungsweges durch die Dialysierflüssigkeitskammer erforderliche Bypassleitung in bekannten Dialysegeräten bereits vorhanden sind, so daß bei diesen Geräten keine umfangreichen Umbauarbeiten notwendig sind. So ist es beispielsweise nicht erforderlich, im Dialysierflüssigkeitskreis Ventilanordnungen vorzusehen, die eine Rezirkulation der Dialysierflüssigkeit durch den Dialysator ermöglichen. Daraus ergibt sich ein vereinfachter Aufbau

Nach dem erfindungsgemäßen Verfahren kann die Blutkonzentration einer Substanz, die am Stoffaustausch im Dialysator teilnimmt), einfach und schnell durch eine kurze Unterbrechung der Dialyse bestimmt werden, indem der im Dialysator verbleibenden Dialysierflüssigkeit gestattet wird, in ein diffuses Gleichgewicht mit dem Blut zu kommen. Nach Ablauf des Gleichgewichtszeitraumes wird dann wieder in den Normalbetrieb geschaltet und die Konzentration der Substanz in der Restmenge der im Dialysator nach dem Abtrennen verbliebenen Dialysierflüssigkeit nach einer gewissen zeitlichen Verzögerung am Ort des ohnehin vorhandenen, stromab angeordneten dialysatseitigen Konzentrationsmeßfühler bestimmt.

Versuche haben gezeigt, daß sich die Gleichgewichtseinstellung sehr schnell vollzieht und im Bereich von wenigen Minuten liegt. Die Unterbrechung der Dialyse für diese sehr kurze Zeitspanne stört daher nicht die Dialysebehandlung und erlaubt auf der anderen Seite eine sehr schnelle und auch einfache dialysatseitige Messung der Blutkonzentration der betrachteten Substanz.

Das Ausspülen der Restmenge der Dialysierflüssigkeit aus der zweiten Kammer kann dadurch erfolgen, daß der Strömungsweg durch die zweite Kammer des Dialysators wieder freigegeben und die Restmenge durch die in die zweite Kammer strömende Dialysierflüssigkeit verdrängt wird. Dadurch läßt sich ein sehr einfaches Meßverfahren erzielen. Es ist aber auch möglich, vor der Freigabe des Dialysierflüssigkeitsweges die Restmenge der Dialysierflüssigkeit aus der zweiten Kammer mittels der Dialysierflüssigkeitspumpe abzuziehen.

Die gemessene Bluteingangskonzentration der betrachteten Substanz kann unmittelbar der zu bestimmmende Parameter der Hämodialyse sein. Sie kann jedoch auch als Zwischenwert für andere zu bestimmenden Parameter der Hämodialyse dienen. So ist es beispielsweise gemäß einer weiteren Ausgestaltung der Erfindung möglich, die Dialysance zu bestimmen, wenn nach dem Abführen der Restmenge aus dem Dialysierflüssigkeits-Kreis und der Bestimmung der Bluteingangskonzentration (cbi) als Zwischenwert zusätzlich nach dem erneuten Einschalten der Dialyse der Dialysierflüssigkeitsfluß (Qd) und die Konzentration der Dialysierflüssigkeit am Ein- und Ausgang des Dialysators (cdi, cdo) gemessen werden, und aus der Gleichung

$$D = Qd \, \frac{(cdo - cdi)}{cbi - cdi}$$

die Dialysance D als Parameter bestimmt wird.

Der vorgegebene Zeitraum für die Gleichgewichtseinstellung liegt vorzugsweise im Bereich von 1 - 3 Minuten. Zur Erhöhung der Genauigkeit ist es bei Zeiten von 1 - 2 Minuten zweckmäßig, anhand des Gradienten der Meßkurve zusätzlich eine Aussage über den zu erwartenden Endwert zu berechnen, um so mit einer kurzen Zeit arbeiten zu können.

Die Konzentranonsmessung erfolgt vorzugsweise, wie üblich, durch eine Leitfähigkeitsmessung.

Ferner liegt der Erfindung die Aufgabe zugrunde, eine Vorrichtung zur Druchführung des Verfahrens nach einem der Ansprüche 1 bis 5 zu schaffen, die eine sehr einfache und schnelle dialysatseitige Messung der Blutkonzentration der am Stoffaustausch des Dialysators teilnehmenden Substanz unmittelbar als der zu bestimmende Parameter oder Zwischenwert für den zu bestimmenden Parameter erlaubt, ohne daß der Patient während der Messung einer möglichen Belastung durch einen Flüssigkeitsentzug ausgesetzt ist.

Die Lösung dieser Aufgabe erfolgt erfindungsgemäß mit den im Patentanspruch 6 angegebenen Merkmalen.

Mit der erfindungsgemäßen Vorrichtung läßt sich auch die Dialysance des Dialysators in-vivo auf sehr einfache und schnelle Weise ermitteln, wenn gemäß einer Weiterbildung der Erfindung auf die Steuer-/Auswerteschaltung zusätzlich ein Signal für den Wert der Konzentration in der Dialysierflüssigkeit stromab und stromauf des Dialysators und ein Signal für den Durchfluß der Dialysierflüssigkeit aufgeschaltet ist und die Zeittaktsteuerung in der Steuer-/Auswerteschaltung so bestimmt ist, daß nach Messung der Bluteingangskonzentration (cbi) und dem Wiedereinschalten des Dialysierflüssigkeitskreises die Signale für den Durchfluß (Qd) sowie für die Eingangs- und Ausgangskonzentrationen (cdi, cdo) in der Dialysierflüssigkeit als Meßsignale ausgewertet werden und nach der Gleichung

$$D = Qd \, \frac{(cdo - cdi)}{cbi - cdi}$$

die Dialysance D bestimmt wird.

Im folgenden wird die Erfindung unter Bezugnahme auf die Zeichnungen näher erläutert.

Es zeigen:

Figur 1    ein Prinzipschaltbild einer Blutbehandlungsvorrichtung mit einer Einrichtung zur Bestimmung von Parametern der Hämodialyse,

Figur 2a      ein Schaubild, in dem das bei einem Dialysator mit einer Membranfläche von 0,7 m$^2$ gemessene Verhältnis von dialysatseitiger Leitfähigkeit und blutseitiger Leitfähigkeit als Funktion der Dialysierflüssigkeitsflußrate dargestellt ist,

Figur 2b      ein Schaubild, in dem das bei einem Dialysator mit einer Membranfläche von 1,8 m$^2$ gemessene Verhältnis von dialysatseitiger Leitfähigkeit und blutseitiger Leitfähigkeit als Funktion der Dialysierflüssigkeitsflußrate dargestellt ist,

Figur 2c      ein Schaubild, in dem die zeitliche Änderung der dialysatseitigen Leitfähigkeit infolge einer Veränderung der Dialysierflüssigkeitsflußrate dargestellt ist,

Figur 3      ein Prinzipschaltbild eines weiteren Ausführungsbeispiels einer Vorrichtung zur in-vivo-Bestimmung von Parametern der Hämodialyse, ohne daß der Patient während der Messung einer möglichen Belastung durch einen Flüssigkeitsentzug ausgesetzt ist und

Figur 4      ein Zeit-Takt-Diagramm für die Einsteuerung des Meßbetriebes in den Dialysebetrieb der Vorrichtung von Figur 3.

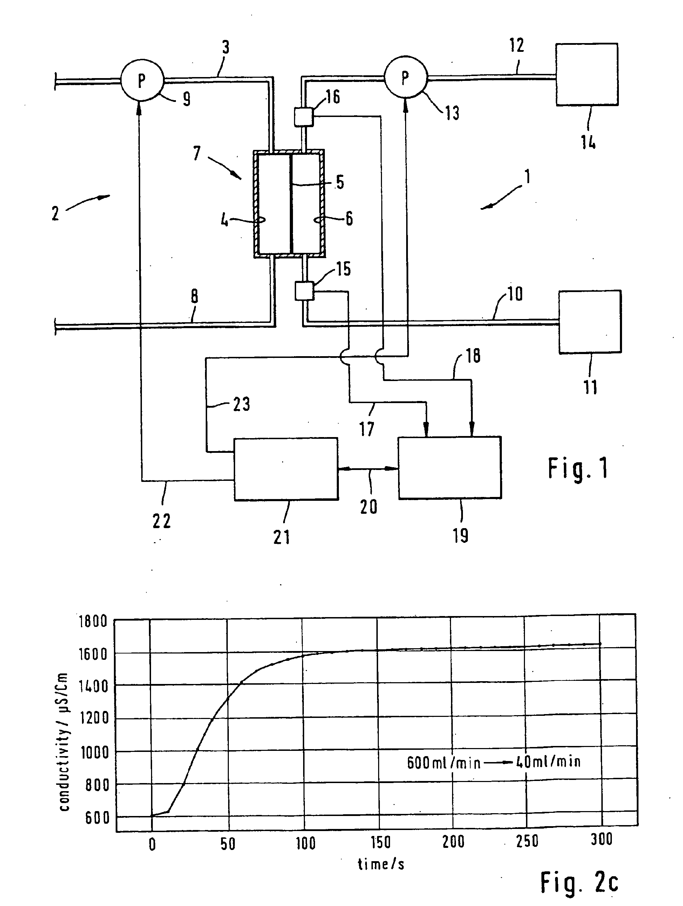

In Figur 1 sind nur die wesentlichen Komponenten einer Dialysevorrichtung dargestellt. Die Dialysevorrichtung besteht im wesentlichen aus einem Dialysierflüssigkeitsteil 1 und einem extrakorporalen Blutkreislauf 2. Der extrakorporale Blutkreislauf 2 umfaßt einen arteriellen Zweig 3, eine Blutkammer 4 eines durch eine semipermeable Membran 5 in die Blutkammer 4 und eine Dialysierflüssigkeitskammer 6 unterteilten Dialysators 7 und einen venösen Zweig 8. Im arteriellen Zweig 3 ist eine Blutpumpe 9 angeordnet, mit der eine bestimmte Blutflußrate Qb im extrakorporalen Kreislauf vorgegeben werden kann.

Die Dialysierflüssigkeitskammer 6 des Dialysators 7 ist über eine Zuführleitung 10 mit einer Dialysierflüssigkeitsquelle 11 verbunden. Eine Abführleitung 12, in die eine die Flußrate Qd der Dialysierflüssigkeit vorgebende Dialysierflüssigkeitspumpe 13 geschaltet ist, führt in einen Auslauf 14.

In der Zuführleitung 10 und der Abführleitung 12 ist jeweils eine Meßeinrichtung 15, 16 zur Bestimmung der Stoffkonzentration der Dialysierflüssigkeit am Eingang des Dialysators 7 und der Stoffkonzentration der Dialysierflüssigkeit am Ausgang des Dialysators angeordnet. Die Meßeinrichtung 15 ist zur Durchführung des erfindungsgemäßen Verfahrens zunächst nicht notwendig. Für die Bestimmung weiterer Parameter ist das Vorhandensein einer solche Meßeinrichtung jedoch vorteilhaft. Die Meßeinrichtungen 15, 16 zur Bestimmung der Dialysierflüssigkeitseingangs- und -ausgangskonzentration cdi bzw. cdo weisen stromauf und stromab des Dialysators 7 angeordnete Leitfähigkeitssensoren auf, die vorzugsweise die temperaturkorrigierte Leitfähigkeit der Dialysierflüssigkeit messen, die hauptsächlich durch die Na-Konzentration bedingt ist. Anstelle von Leitfähigkeitssensoren können auch optische Sensoren oder Enzymsensoren etc. zur Messung der Dialysierflüssigkeitskonzentration im Dialysierflüssigkeitsweg 1 angeordnet sein. Anstelle eines Leitfähigkeitssensors kann die Meßeinrichtung 16 stromab des Dialysators 7 auch einen Harnstoffsensor aufweisen, wobei ein Harnstoffsensor stromauf des Dialysators entfallen kann, wenn die Clearance bestimmt werden soll. Die Meßeinrichtungen 15, 16 sind über Datenleitungen 17, 18 mit einer Auswerteinheit 19 verbunden, in der die Ausgangssignale der Meßeinrichtungen verarbeitet werden. Die Auswerteinheit kommuniziert über eine Datenleitung 20 mit einer Steuereinheit 21. Die Steuereinheit 21 ist über Steuerleitungen 22, 23 mit der Blutpumpe 9 und der Dialysierflüssigkeitspumpe 13 verbunden, so daß die Blutflußrate Qb bzw. die Dialysierflüssigkeitsflußrate Qd eingestellt und variiert werden kann.

Während der Dialysebehandlung strömt die in der Dialysierflüssigkeitsquelle 11 bereitgestellte Dialysierflüssigkeit durch die Dialysierflüssigkeitskammer 6 des Dialysators 7 mit einer durch die Fördermenge der Dialysierflüssigkeitspumpe 13 vorgegebenen Flußrate Qd, während das zu behandelnde Blut des Patienten die Blutkammer 4 des Dialysators 7 mit einer durch die Blutpumpe 9 vorgegebenen Flußrate Qb durchströmt.

Der von der Steuereinheit 21 vorgegebene Programmablauf zur Bestimmung der Bluteingangskonzentration cbi wird nachfolgend erläutert.

Während der Hämodialysebehandlung wird die Dialysierflüssigkeitsflußrate auf 40 bis 60 ml/min, vorzugsweise 50 ml/min, reduziert. Die Reduzierung der Flußrate erfolgt dadurch, daß die Steuereinheit 21 die Dialysierflüssigkeitspumpe 13 entsprechend ansteuert. Nach Ablauf einer Zeitdauer von 2 bis 3 Minuten, die von einem in der Steuereinheit vorgesehenen Zeitglied vorgegeben wird, steuert die Steuereinheit die Auswerteinheit 19 derart an, daß die Auswerteinheit die mit der Meßeinrichtung 16 gemessene Dialysierflüssigkeitsausgangskonzentration cdo erfaßt. Die gemessene Dialysierflüssigkeitsausgangskonzentration cdo entspricht dann im wesentlichen der Bluteingangskonzentration cbi als den zu bestimmenden Parameter. Anschließend stellt die Steuereinheit 21 mit der Dialysierflüssigkeitspumpe 13 die Dialysierflüssigkeitsflußrate wieder auf den ursprünglichen Wert ein.

Die ermittelte Bluteingangskonzentration cbi kann als Zwischenwert für die Ermittlung weiterer Parameter herangezogen werden. So läßt sich die Dialysance des Dialysators 7 durch zwei weitere Messungen ermitteln. Hierzu steuert die Steuereinheit 21 die Auswerteinheit 19 derart an, daß diese die bei einem vorgegebenen Dialysierflüssigkeitsfluß Qd mit der Meßeinrichtung 15 gemessene Dialysierflüssigkeitseingangskonzentration cbi und die sich nunmehr einstellende Dialysierflüssigkeitsausgangskonzentration cdo, die mit der Meßeinrichtung 16 gemessen wird, erfaßt. In der Auswerteinheit wird aus der zuvor bestimmten Bluteingangskonzentration cdi und der Dialysierflüssigkeitseingangs- und - ausgangskonzentration cdi, cdo sowie der vorgegebenen Dialysierflüssigkeitsrate Qd nach der Gleichung

$$D = Qd \bullet \frac{cdo - cdi}{cbi - cdi} \qquad (3)$$

die Dialysance D bestimmt.

Die zu bestimmenden Parameter können auf einer nicht dargestellten Anzeigeeinheit ausgegeben bzw. zur weiteren Prozeßsteuerung herangezogen werden.

Das erfindungsgemäße Verfahren beruht darauf, daß bei einer Reduzierung der Flußrate der Dialysierflüssigkeit trotz variabler Behandlungsbedingungen die Bluteingangskonzentration dadurch bestimmt werden kann, daß die Dialysierflüssigkeitsausgangskonzentration gemessen wird. Dies hat den Vorteil, daß ein Eingriff in den extrakorporalen Blutkreislauf nicht erforderlich ist. Die Genauigkeit des Meßverfahrens hat sich in den nachfolgenden Experimenten bestätigt.

Es wurde mittels zweier Schlauchrollenpumpen der gekoppelte Dialysat- und Blutkreislauf nachgebildet. Da Diffüsionsverhalten und Dialysator-Clearances für Elektrolyte und für Harnstoff nahezu identisch sind, konnte anstelle von Harnstoff NaCl als Testsubstanz verwendet werden. Dadurch ist über die Leitfähigkeitsmessung eine einfache, hochgenaue Methode zur Konzentrationsmessung verfügbar. Blutseitig wurde eine 15 millimolare NaCl-Lösung verwendet. Diese Konzentration liegt in der Größenordnung der typischen Harnstoffkonzentration. Dialysatseitig wurde reines Wasser verwendet. Die Leitfähigkeitsmeßzellen wurden stromauf und stromab der Dialysierflüssigkeitskammer des Dialysators angeordnet.

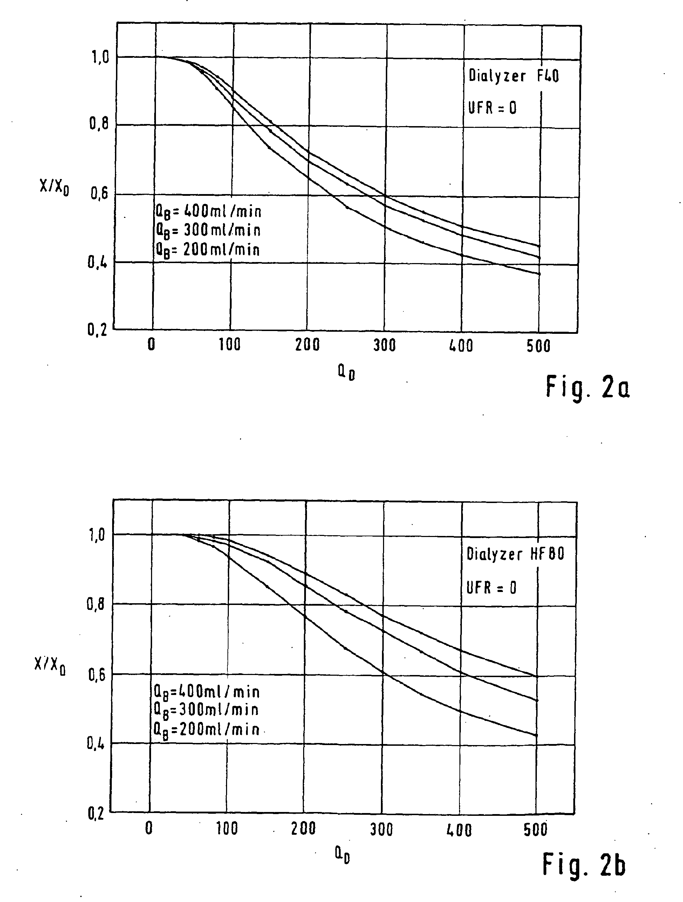

Die Messungen wurden mit verschiedenen Dialysatortypen durchgeführt, die sich in ihrer Membranfläche voneinander unterscheiden. Für jeden Dialysator wurden Meßkurven für einige Blutflüsse aufgenommen. Als minimaler Blutfluß, der klinisch üblich ist, wurde 200 ml/min angesetzt. Für einen gegebenen Blutfluß Qb wurde der Dialysatfluß Qd schrittweise von 500 ml/min bis 0 ml/min verändert, wobei nach jeder Veränderung des Dialysatflusses Qd die Einstellung eines stabilen Zustandes abgewartet und dann das Verhältnis der dialysatseitigen zur blutseitigen Leitfähigkeit bestimmt wurde. Figur 2a zeigt das Verhältnis $X/X_0$ der dialysatseitigen Leitfähigkeit zur blutseitigen Leitfähigkeit als Funktion der Dialysierflüssigkeitsflußrate Qd bei einem Dialysator mit einer Membranfläche von 0,7 m$^2$, während Figur 2b das Verhältnis von dialysatseitiger zur blutseitiger Leitfähigkeit als Funktion der Dialysierflüssigkeitsflußrate bei einem Dialysator mit einer Membranfläche von 1,8 m$^2$ zeigt. Die dialysatseitige Leitfähigkeit bzw. blutseitige Leitfähigkeit entspricht der Dialysierflüssigkeitsausgangs- bzw. Bluteingangskonzentration. Wie die Messungen zeigen, wird das Gleichgewicht bei einer Dialysierflüssigkeitsflußrate Qd von 50 ml/min nahezu vollständig erreicht. Bei dem Dialysator mit einer Membranfläche von 0, 7 m$^2$ ist das Verhältnis $X/X_0 > 0,97$ für Qb $\geq$ 200 ml/min und bei einem Dialysator mit einer Membranfläche von 1,8 m$^2$ ist $X/X_0 > 0,99$ für Qb $\geq$ 200 ml/min. Bei höheren Blutflüssen ergibt sich ein noch besserer Konzentrationsausgleich.

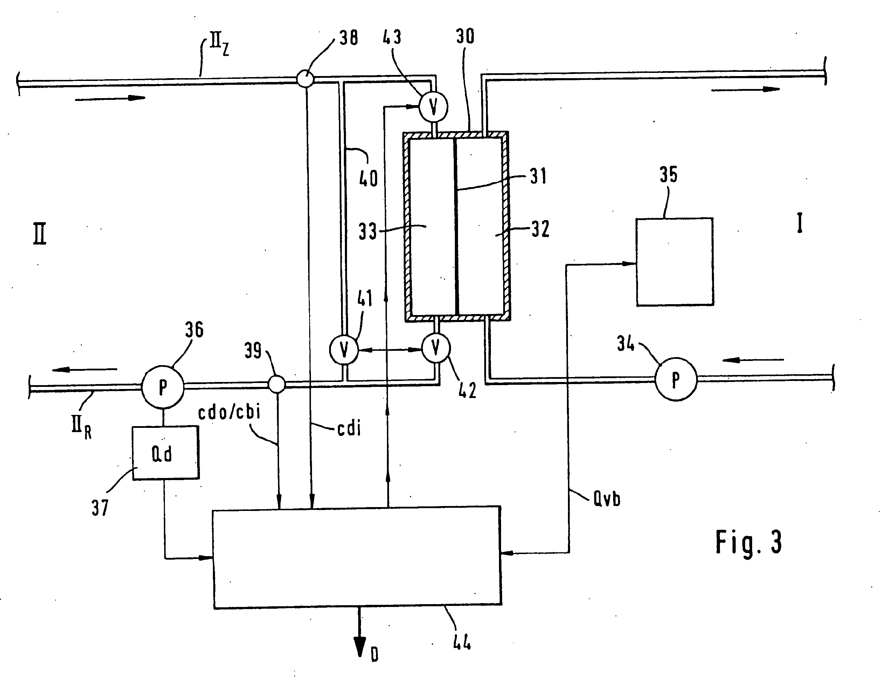

Figur 3 zeigt ein Prinzipschaltbild der erfindungsgemäßen Vorrichtung, mit der das erfindungsgemäße Verfahren zur in-vivo-Bestimmung von Parametern der Hämodialyse durchgeführt wird, ohne daß der Patient während der Messung einer möglichen Belastung durch einen Flüssigkeitsentzug ausgesetzt ist.

Die Vorrichtung weist einen Dialysator 30 mit einer semipermeablen Membran 31 auf, die eine Blutkammer 32 von einer Dialysatkammer 33 trennt. Die Blutkammer 32 ist an einen extrakorporalen Kreislauf I angeschlossen, in dem das zu reinigende Blut eines Dialysepatienten mit einer durch eine Blutpumpe 34 vorgegebenen Flußrate fließt. Mit 35 ist eine Einrichtung bezeichnet, mittels der die Drehzahl der Blutpumpe 34 und damit der Vollblutfluß Qvb geändert werden kann. Solche Einrichtungen sind Stand der Technik, wie im übrigen der weitere Aufbau des extrakorporalen Kreislaufes I Stand der Technik ist und daher in dem Prinzipschaltbild nach der Zeichnung nicht dargestellt ist.

Die Dialysatkammer 33 ist an einen Dialysatkreislauf II mit konventionellem Aufbau angeschlossen, von dem der Übersicht halber nur eine in die Rückführleitung II$_R$ geschaltete Dialysatpumpe 36 mit einer zugehörigen Einrichtung 37 zum Ändern der Drehzahl dieser Pumpe und die in die Zuführleitung II$_Z$ bzw. die Rückführleitung des Dialysatkreislaufs II geschalteten Leitfähigkeitssensoren 38 und 39 dargestellt sind. Die Leitfähigkeitssensoren messen vorzugsweise die temperaturkorrigierte Leitfähigkeit der Dialysierflüssigkeit auf der Basis der Na-Konzentration. Anstelle der Bestimmung der Leitfähigkeit kann die Konzentrationsmessung auch über die Messung von entsprechenden optischen Eigenschaften erfolgen.

Die Vorrichtung weist ferner eine Bypassleitung 40 sowie ein Bypassventil 41 und zwei Dialysator-Abschaltventile 42, 43 auf.

Der übrige Aufbau ist bekannt, wozu beispielsweise auf die eingangs zitierte EP 0 097 366 hingewiesen wird. Die Dialysierflüssigkeit durchströmt im Dialysebetrieb die Dialysatkammer 33 mit einer durch die Drehzahl der Pumpe 36 vorgegebenen Flußrate Qd sowie einer durch die nicht dargestellte Konzentratmischung vorgegebenen Eingangskonzentration cdi, die mittels des stromauf angeordneten Leitfähigkeitssensors 38 erfaßt wird. Die sich bei der Dialyse einstellende Ausgangskonzentration cdo wird mittels des stromab angeordneten Leitfähigkeitssensor 39 erfaßt. Aus der Differenz cdi - cdo kann dann der Elektrolyttransfer berechnet werden. Prinzipiell kann der Leitfähigkeitssensor 38 entfallen und der Meßwert durch einen eingestellten, d.h. vorgegebenen Wert von cdi ersetzt werden. Durch Abschalten der Ventile 42, 43 kann der Dialysator 1 von dem Dialysatkreis II abgetrennt werden, wobei die Dialysierflüssigkeit durch Öffnen des Ventils 41 über die Bypassleitung 40 am Dialysator vorbeifließt.

Alle Signale für die Flüsse Qb und Qd sowie für die dialysatseitigen Konzentrationen cdi und cdo werden einer Steuer-/Auswertestufe 44 zugeführt, die vorzugsweise durch einen Mikroprozessor gebildet wird, der in der Regel ohnehin in einem Dialysegerät vorhanden ist. In dieser Auswertestufe 44 werden die Signale miteinander verknüpft, um gewünschte Parameter der Hämodialyse zu bestimmen. So kann beispielsweise in dieser Stufe 44 die Elektrolyttransferrate Qd x (cdi - cdo) errechnet und mit anderen Größen u.a. auf der Basis der Massenbilanz im Dialysator in Beziehung gesetzt werden. Diese Stufe 44 ist ferner über Steuerleitungen mit den Ventilen 41, 42, 43 verbunden.

Die Basis für die erfindungsgemäße in-vivo-Bestimmung von Parametern der Hämodialyse ist die Bestimmung der Konzentration einer am Stoffaustausch des Dialysators 30 teilnehmenden Substanz im Blut des Dialysepatienten, die sogenannte Bluteingangskonzentration cbi. Diese Bluteingangskonzentration kann der zu bestimmende Parameter selbst sein. Sie kann aber auch als Zwischenwert für den zu bestimmenden Parameter dienen.

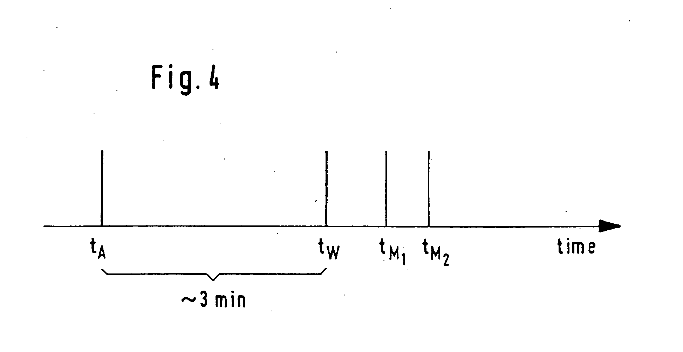

Zur Bestimmung des Wertes cbi erzeugt ein Steuerteil in der Steuer/Auswertestufe, manuell aktiviert durch die Bedienungsperson oder programmgesteuert, zum Zeitpunkt $t_A$ (Fig. 4) ein Schaltsignal an die Ventilanordnung 41, 42, 43 derart, daß der Dialysator 1 durch Schließen der Ventile 42, 43 vom Dialysatkreis II getrennt wird und die Bypaßleitung 40 durch Öffnen des Ventiles 41 eingeschaltet wird. Der Blutfluß im extrakorporalen Kreislauf I wird dabei nicht angehalten. Die in der Dialysatkammer 33 verbleibende Restmenge an Dialysierflüssigkeit, bei üblichen Dialysatoren eine Menge von deutlich mehr als 100 ml Dialysierflüssigkeit, wird nun eine vorgegebene Zeit dem Stoffaustausch mit dem in der Blutkammer strömenden Blut ausgesetzt, bis sich in den Konzentrationen der Flüssigkeiten in beiden Kammern ein Gleichgewicht eingestellt hat, d.h. die Restmenge an Dialysat in der Dialysatkammer die Bluteingangskonzentration cbi bzw. die Eingangsleitfähigkeit des Blutes angenommen hat. Da die Dialysatkammer des Dialysators ein vollständig abgeschlossenes Volumen bildet, findet während der Messung eine Ultrafiltration nicht statt. Wie Versuche gezeigt haben, ist bereits nach ca. 3 Minuten dieser Gleichgewichtszustand erreicht. Nach Ablauf dieser Zeit erzeugt zum Zeitpunkt $t_W$ in Fig. 2 der vorerwähnte Steuerteil in der Stufe 44 durch eine Zeitschaltung ein weiteres Signal, welches das Bypaßventil 41 schließt und damit die Bypaßleitung 40 abschaltet sowie welches die Ventile 42, 43 öffnet und damit den Dialysator wieder an den Dialysatkreis anschaltet. Die frisch zuströmende Dialysierflüssigkeit drückt nun die Restmenge in der Dialysatkammer 33, die den Wert cbi angenommen hat, aus dieser Kammer heraus und führt sie an der Leitfähigkeitsmeßzelle 39 vorbei. Es wird dann nur noch zum richtigen Zeitpunkt $t_{M1}$, wenn diese Restmenge an der stromab gelegenen Leitfähigkeitsmeßzelle 39 vorbeiströmt, die Leitfähigkeit (und damit die Konzentration) gemessen und damit cbi bestimmt. Der richtige Zeitpunkt $t_{M1}$ wird dadurch bestimmt, daß im offenen Zustand des Dialysatkreises ohne Bypaß über die Korrelationsfunktion (bestimmt durch Entfernung der Leitfähigkeitsmeßzelle vom Dialysator und durch den Dialysatdurchfluß) die Dialysatlaufzeit von dem Dialysator 30 bis zum Ort der Leitfähigkeitmeßzelle 39 ermittelt wird.

Mißt man nach Öffnen des Bypasses 40 und nach dem Entfernen der vorgegebenen Restmenge aus dem Dialysatkreis II zum Zeitpunkt $t_{M2}$ zusätzlich stromauf und stromab die Konzentrationen cdi und cdo in der Dialysierflüssigkeit sowie den Dialysatfluß Qd, dann stehen alle Meßgrößen zur Verfügung, um in der Stufe 44 gemäß der Gleichung (3) die Dialysance D bestimmen zu können.

Unter Einbeziehung des Signals über den Vollblutfluß Qvb lassen sich weitere Parameter der Hämodialyse zumindest abschätzen.

**Patentansprüche**

1. Verfehren zur in-vivo-Bestimmung von Parametern der Hämodialyse auf der Basis der Bestimmung der Konzentration einer Substanz in einem extrakorporalen Blutkreislauf, in den eine erste Kammer eines Dialysators geschaltet ist, die mittels einer semipermeablen Membran von einer zweiten, an einen Dialysierflüssigkeits-Kreis angeschlossenen Kammer getrennt ist, wobei

   der Dialysierflüssigkeitsfluß durch die zweite Kammer des Dialysators für einen vorgegebenen Zeitraum, in dem die Dialysierflüssigkeit zumindest innerhalb bestimmter Toleranzen die Eingangskonzentration der betrachteten Substanz in dem durch den extrakorporalen Blutkreislauf strömenden Blut zumindest in einem Teilvolumen der zweiten Kammer angenommen hat, ohne Unterbrechung des Blutflusses unterbrochen wird und die Konzentration der Substanz in der Dialysierflüssigkeit nach der Einstellung des Gleichgewichtszustandes als der zu bestimmende Parameter oder als Zwischenwert für den zu bestimmenden Parameter gemessen wird, wobei die Restmenge der Dialysierflüssigkeit in der zweiten Kammer des Dialysators nach der Einstellung des Gleichgewichtszustandes aus der zweiten Kammer ausgespült und die Konzentration der Substanz in der aus der zweiten Kammer strömenden Restmenge der Dialysierflüssigkeit dann stromab des Dialysators gemessen wird.

2. Verfahren nach Anspruch 1, **dadurch gekennzeichnet, daß** eine den Strömungsweg durch die zweite Kammer des Dialysators überbrückende Bypassleitung in den Dialysierflüssigkeits-Kreis geschaltet wird und der Zufluß von Dialysierflüssigkeit in die zweite Kammer des Dialysators und der Abfluß von Dialysierflüssigkeit aus der zweiten Kammer des Dialysators unterbrochen wird, so daß die zweite Kammer des Dialysators nicht mehr durchströmt und Flüssigkeit aus der zweiten Kammer nicht abgezogen wird.

3. Verfahren nach Anspruch 1 oder 2, **dadurch gekennzeichnet, daß** nach dem Ausspülen der Restmenge der Dialysierflüssigkeit aus der zweiten Kammer des Dialysators und der Bestimmung der Bluteingangskonzentration (cbi) als Zwischenwert zusätzlich nach der Freigabe des Strömungsweges durch die zweite Kammer des Dialysators der Dialysierflüssigkeitsfluß (Qd) und die Konzentration der Dialysierflüssigkeit am Ein- und Ausgang des Dialysators (cdi, cdo) gemessen werden, und aus der Gleichung

$$D = Qd\ \frac{(cdo - cdi)}{cbi - cdi}$$

die Dialysance D als Parameter bestimmt wird.

4. Verfahren nach einem der Ansprüche 1 bis 3, **dadurch gekennzeichnet, daß** der vorgegebene Zeitraum für die Gleichgewichtseinstellung im Bereich von 1 - 3 min. liegt.

5. Verfahren nach einem der Ansprüche 1 bis 4, **dadurch gekennzeichnet, daß** die Konzentranonsmessung durch eine Leitfähigkeitsmessung erfolgt.

6. Vorrichtung zur Durchführung des Verfahrens nach einem der Ansprüche 1 bis 5, mit einem Dialysator (30), der eine semipermeable Membran (31) ausweist, die eine Blutkammer (32) von einer Dialysatkammer (33) trennt, von denen die Blutkammer (32) über einen extrakorporalen Kreislauf (I) an einen Dialyse-Patienten abschließbar ist, und von denen die Dialysatkammer (33) an einen Dialysierflüssigkeits-Kreis (II) angeschlossen ist, der zumindest stromab des Dialysators (30) einen Konzentrationsmeßfühler (39) zur Messung der Konzentration in der Dialysierflüssigkeit am Ausgang des Dialysators (1) aufweist, und mit einer Einrichtung (42, 43) zum Unterbrechen des Dialysierflüssigkeitsflusses durch den Dialysator (30), mit der eine Steuer/Auswerteschaltung (44) verbunden ist, auf die zumindest das Ausgangssignal des Konzentrationsmeßfühlers (39) geschaltet ist, und die derart ausgebildet ist, daß die Steuer/Auswertschaltung (44) zum Zeitpunkt der Basis-Bestimmung der Konzentration der Substanz im Blut des Patienten ein Signal für das Abtrennen der Dialysatkammer (33) an die zugehörige Einrichtung zum Unterbrechen des Dialysierflüssigkeitsflusses (42, 43) abgibt, so daß die zweite Kammer des Dialysators nicht durchströmt und Flüssigkeit aus der zweiten Kammer nicht abgezogen wird, und nach einem vorgegebenen Zeitraum, in dem zumindest ein Teil der in der Dialysatkammer (33) nach dem Abtrennen verbliebenen, Restmenge der Dialysierflüssigkeit zumindest innerhalb bestimmter Toleranzen die Eingangskonzentration der betrachteten Substanz im Blut angenommen hat, ein Signal zum Wiedereinschalten des Dialysators an die zugehörige Einrichtung zum Unterbrechen des Dialysierflüssigkeitsflusses (42, 43) abgibt, und dann die nach einem Zeitraum, der von dem Durchfluß der Dialysierflüssigkeit und der Entfernung zwischen Ausgang des Dialysators (30) und dem Ort des Konzentrationsmeßfühlers (39) bestimmt ist, das Ausgangssignal des Konzentrationsmeßfühlers (39) als Meßsignal für die Konzentration der Substanz in der Restmenge der Dialysierflüssigkeit nach der Einstellung des Gleichgewichtszustandes für die Bestimmung der Eingangskonzentration der betrachteten Substanz im Blut auswertet.

7. Vorrichtung nach Anspruch 6, **dadurch gekennzeichnet, daß** eine Bypassleitung (40) vorgesehen ist, mit der die Dialysatkammer (33) des Dialysators (30) überbrückbar ist, wenn der Dialysierflüssigkeitsfluß durch den Dialysator unterbrochen ist.

**8.** Vorrichtung nach Anspruch 6 oder 7, dadurch gekennzeichet, daß auf die Steuer-/Auswerteschaltung (44) zusätzlich ein Signal für den Wert der Konzentration in der Dialysierflüssigkeit stromab und stromauf des Dialysators (30) und ein Signal für den Durchfluß der Dialysierflossigkeit aufgeschaltet ist und die Zeittaktsteuerung in der Steuer-/ Auswerteschaltung (44) so bestimmt ist, daß nach Messung der Bluteingangskonzentration (cbi) und dem Wiedereinschalten des Dialysierflüssigkeitskreises die Signale für den Durchfluß (Qb) sowie für die Eingangs- und Ausgangskonzentrationen (cdi, cdo) in der Dialysierflüssigkeit als Meßsignale ausgewertet werden und nach der Gleichung

$$D = Qd \frac{(cdo - cdi)}{cbi - cdi}$$

die Dialysance D bestimmt wird.

**9.** Vorrichtung nach einem der Ansprüche 6 bis 8, dadurch gekenntzeichnet, daß als Konzentrationsfühler (38, 39) Leitfähigkeitsmeßfühler oder optische Meßfühler vorgesehen sind.

**Claims**

**1.** A method for determining in-vivo hemodialysis parameters on the basis of determining the concentration of a substance in an extracorporeal blood circuit to which a first chamber of a dialyzer is connected, which first chamber is separated by means of a semipermeable membrane from a second chamber that is connected to a dialysate circuit, wherein
the dialysate flow through the second chamber of the dialyzer is interrupted, without interrupting the blood flow, for a predefined time period in which the dialysate has adopted, at least within certain tolerances, the incoming concentration of the observed substance in the blood flowing through the extracorporeal blood circuit at least in a partial volume of the second chamber, and
after the state of equilibrium is set, the concentration of the substance in the dialysate is measured as the parameter to be determined or as an intermediate value for the parameter to be determined,
wherein
the remaining quantity of dialysate in the second chamber of the dialyzer is flushed out after the state of equilibrium is set, and the concentration of the substance in the remaining quantity of the dialysate flowing out of the second chamber is then measured downstream of the dialyzer.

**2.** The method according to claim 1, **characterized in that** a bypass line bridging the flow path through the second chamber of the dialyzer is connected to the dialysate circuit, and the inflow of dialysate into the second chamber of the dialyzer and the outflow of dialysate from the second chamber of the dialyzer is interrupted so that the flow no longer passes through the second chamber of the dialyzer and no fluid is extracted from the second chamber.

**3.** The method according to claim 1 or claim 2, **characterized in that** after flushing the dialysate out of the second chamber of the dialyzer and determining the incoming blood concentration (cbi) as an intermediate value, the dialysate flow (Qd) and the concentration of the dialysate at the inlet and the outlet of the dialyzer (cdi, cdo) are additionally measured after opening the flow path through the second chamber of the dialyzer, and the dialysance D is determined as a parameter using the equation

$$D = Qd \frac{(cdo - cdi)}{cbi - cdi}.$$

**4.** The method according to any one of the claims 1 to 3, **characterized in that** the predefined time period for setting the equilibrium is in the range of from 1 - 3 min.

5. The method according to any one of the claims 1 to 4, **characterized in that** the concentration measurement is carried out through a conductivity measurement.

6. A device for carrying out the method according to any one of the claims 1 to 5, comprising a dialyzer (30) having a semipermeable membrane (31) that separates a blood chamber (32) from a dialysate chamber (33), the blood chamber (32) being connectable to a dialysis patient via an extracorporeal circuit (I), and the dialysate chamber (33) being connected to a dialysate circuit (II) which, at least downstream of the dialyzer (30), has a concentration sensor (39) for measuring the concentration in the dialysate at the outlet of the dialyzer (1), and comprising a mechanism (42, 43) for interrupting the dialysate flow through the dialyzer, (30) to which mechanism a control/analyzer circuit (44) is connected and to which at least the output signal of the concentration sensor (39) is transmitted, and which is configured such that at the time of the basis determination of the concentration of the substance in the blood of the patient, the control/analyzer circuit (44) emits a signal to the associated mechanism (42, 43) for interrupting the dialysate flow so as to disconnect the dialysate chamber (33) so that the flow does not pass through the second chamber of the dialyzer and fluid is not extracted from the second chamber and, after a predefined time period in which at least a portion of the quantity of dialysate remaining in the dialysate chamber (33) after disconnecting has adopted the incoming concentration of the monitored substance in the blood at least within certain tolerances, emits a signal to the associated mechanism (42, 43) for interrupting the dialysate flow so as to connect the dialyzer again, and then, after a time period that is determined by the flow rate of the dialysate and the distance between the outlet of the dialyzer (30) and the location of the concentration sensor (39), analyzes the output signal of the concentration sensor (39) as measurement signal for the concentration of the substance in the remaining quantity of dialysate after the equilibrium state is set so as to determine the incoming concentration of the observed substance in the blood.

7. The device according to claim 6, **characterized in that** a bypass line (40) is provided through which the dialysate chamber (33) of the dialyzer (30) can be bridged when the dialysate flow through the dialyzer is interrupted.

8. The device according to claim 6 or claim 7, **characterized in that** a signal for the value of the concentration in the dialysate downstream and upstream of the dialyzer (30) and a signal for the flow rate of the dialysate is additionally transmitted to the control/analyzer circuit (44), and the time interval control in the control/analyzer circuit (44) is determined such that after measuring the incoming blood concentration (cbi) and reconnecting the dialysate circuit, the signals for the flow rate (Qb) and for the incoming and outgoing concentrations (cdi, cdo) in the dialysate are analyzed as measurement signals, and the dialysance D is determined using the equation

$$D = Qd\,\frac{(cdo - cdi)}{cbi - cdi}.$$

9. The device according to any one of the claims 6 to 8, **characterized in that** conductivity sensors or optical sensors are provided as concentration sensors (38, 39).

## Revendications

1. Procédé pour la détermination in vivo de paramètres de l'hémodialyse sur la base de la détermination de la concentration d'une substance dans un circuit sanguin extracorporel, dans lequel une première chambre d'un dialyseur, qui est séparée au moyen d'une membrane semi-perméable d'une deuxième chambre raccordée à un circuit de liquide de dialyse , est mise en service, où
le flux de liquide de dialyse à travers la deuxième chambre du dialyseur est interrompu pendant une durée prédéterminée pendant laquelle le liquide de dialyse a présenté, au moins dans les limites de tolérances déterminées, la concentration d'entrée de la substance considérée dans le sang circulant dans le circuit sanguin extracorporel au moins dans un volume partiel de la deuxième chambre, sans interruption du flux sanguin et
la concentration de la substance dans le liquide de dialyse est mesurée après l'établissement de l'état d'équilibre comme paramètre destiné à être déterminé ou comme valeur intermédiaire pour le paramètre destiné à être déterminé,

où

la quantité résiduelle de liquide de dialyse dans la deuxième chambre du dialyseur est évacuée de la deuxième chambre après l'établissement de l'état d'équilibre et la concentration de la substance dans la quantité résiduelle du liquide de dialyse quittant la deuxième chambre est ensuite mesurée en aval du dialyseur.

2. Procédé selon la revendication 1 **caractérisé en ce qu'** un conduit de bypass contournant le trajet d'écoulement à travers la deuxième chambre du dialyseur est mis en service dans le circuit de liquide de dialyse et le flux entrant de liquide de dialyse dans la deuxième chambre du dialyseur et le flux sortant de liquide de dialyse de la deuxième chambre du dialyseur sont interrompus, de sorte que la deuxième chambre du dialyseur n'est plus traversée et du liquide n'est pas retiré de la deuxième chambre.

3. Procédé selon la revendication 1 ou 2 **caractérisé en ce que**, après le retrait de la quantité résiduelle de liquide de dialyse de la deuxième chambre du dialyseur et la détermination de la concentration sanguine à l'entrée (cbi) comme valeur intermédiaire, en outre, après la libération du trajet d'écoulement à travers la deuxième chambre du dialyseur le flux de liquide de dialyse (Qd) et la concentration du liquide de dialyse à l'entrée et à la sortie du dialyseur (cdi, cdo) sont mesurés, et la dialysance D est déterminée comme paramètre d'après l'égalité

$$D = Qd \frac{(cdo - cdi)}{cbi - cdi}$$

4. Procédé selon l'une des revendications 1 à 3 **caractérisé en ce que** la durée prédéterminée pour l'établissement de l'équilibre est située dans le domaine de 1 - 3 min.

5. Procédé selon l'une des revendications 1 à 4 **caractérisé en ce que** la mesure de la concentration a lieu par une mesure de conductivité.

6. Dispositif pour la mise en oeuvre du procédé selon l'une des revendications 1 à 5 avec un dialyseur (30), qui comporte une membrane semi-perméable (31) qui sépare une chambre de sang (32) d'une chambre de dialysat (33), parmi lesquelles la chambre de sang (32) peut être raccordée à un patient de dialyse par le biais d'un circuit extracorporel (I), et parmi lesquelles la chambre de dialysat (33) est raccordée à un circuit de liquide de dialyse (II) qui comporte au moins en aval du dialyseur (30) un capteur de mesure de concentration (39) pour la mesure de la concentration dans le liquide de dialyse à la sortie du dialyseur (1), et avec un dispositif (42, 43) pour interrompre le flux de liquide de dialyse à travers le dialyseur (30), auquel est relié un circuit de commande/évaluation (44), auquel au moins le signal de sortie du capteur de mesure de concentration (39) est transmis, et qui est agencé de telle manière que le circuit de commande/évaluation (44) délivre, au moment de la détermination de base de la concentration de la substance dans le sang du patient, un signal pour la séparation de la chambre de dialysat (33) au dispositif associé pour l'interruption du flux de liquide de dialyse (42, 43), de sorte que la deuxième chambre du dialyseur n'est pas traversée et du liquide n'est pas retiré de la deuxième chambre, et après une durée prédéterminée, pendant laquelle au moins une partie de la quantité résiduelle de liquide de dialyse restant dans la chambre de dialysat (33) après la séparation a présenté au moins dans les limites de tolérances déterminées la concentration à l'entrée de la substance considérée dans le sang, délivre un signal pour la remise en service du dialyseur avec le dispositif associé pour l'interruption du flux de liquide de dialyse (42, 43), puis après une durée qui est déterminée par le flux traversant du liquide de dialyse et la distance entre la sortie du dialyseur (30) et l'emplacement du capteur de mesure de concentration (39), exploite le signal de sortie du capteur de mesure de concentration (39) comme signal de mesure pour la concentration de la substance dans la quantité résiduelle du liquide de dialyse après l'établissement de l'état d'équilibre pour la détermination de la concentration à l'entrée de la substance considérée dans le sang.

7. Dispositif selon la revendication 6 **caractérisé en ce qu'** il est prévu un conduit de bypass (40) avec lequel la chambre de dialysat (33) du dialyseur (30) peut être contournée quand le flux de liquide de dialyse dans le dialyseur est interrompu.

8. Dispositif selon la revendication 6 ou 7 **caractérisé en ce qu'** un signal pour la valeur de la concentration dans le liquide de dialyse en aval et en amont du dialyseur (30) et un signal pour le flux traversant du liquide de dialyse sont appliqués en outre au circuit de commande/évaluation (44) et la commande par impulsions dans le circuit de commande/évaluation (44) est déterminée de telle manière qu' après la mesure de la concentration sanguine à l'entrée (cbi) et la remise en service du circuit de liquide de dialyse les signaux pour le flux traversant (Qb) ainsi que pour les concentrations à l'entrée et à la sortie (cdi, cdo) dans le liquide de dialyse sont exploités comme signaux de mesure et la dialysance D est déterminée selon l'égalité

$$D = Qd \frac{(cdo - cdi)}{cbi - cdi}$$

9. Dispositif selon l'une des revendications 6 à 8 **caractérisé en ce que** des capteurs de mesure de conductivité ou des capteurs de mesure optiques sont prévus comme capteurs de concentration (38, 39).

Fig. 1

Fig. 2c

Fig. 2a

Fig. 2b

Fig. 3

II_Z  38  43  30

31

33  32

35

I

II

40

41

36  39

42

P

Qd

37

cdo/cbi

cdi

Qvb

II_R

34

P

44

D

EP 1 927 370 B1

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0291421 B1
- DE 3938662 C2
- EP 0428927 A1
- EP 0330892 A1
- EP 0547025 A
- US 5110477 A
- WO 9409351 A
- EP 0578585 B1
- WO 9408641 A
- EP 0097366 A

**In der Beschreibung aufgeführte Nicht-Patentliteratur**

- Principles and biophysics of dialysis. **SARGENT'' J.A. ; GOTCH. F.A.** Replacement of Renal Function. Nijhoff, Den Haag, 1983
- Clinical Validation of a Predictive Modeling Equation for Sodium. **NGUYEN-KHOA MAN et al.** Artificial Organs. Raven Press, 1985, vol. 9, 150-154